# EUROPÄISCHE PATENTSCHRIFT

(11) **EP 3 157 448 B1**
(45) Veröffentlichungstag und Bekanntmachung des Hinweises auf die Patenterteilung: **20.06.2018**
(21) Anmeldenummer: 16729179.8
(22) Anmeldetag: 27.04.2016
(51) Int. Cl.: A61B 17/34, A61F 9/007

(54) **VORRICHTUNG ZUM EINFÜHREN EINES MEDIUMS ODER EINES INSTRUMENTS IN DEN MENSCHLICHEN KÖRPER, INSBESONDERE PORT ODER TROKAR FÜR DAS MENSCHLICHE AUGE**
DEVICE FOR INTRODUCING A MEDIUM OR INSTRUMENT INTO THE HUMAN BODY, IN PARTICULAR A PORT OR TROCAR FOR THE HUMAN EYE
DISPOSITIF POUR L'INTRODUCTION D'UN SUPPORT OU D'UN INSTRUMENT DANS LE CORPS HUMAIN, EN PARTICULIER PORT OU TROCART POUR L' OEIL HUMAIN

(30) Priorität: 19.06.2015 DE 102015211343; 30.06.2015 DE 102015212159; 30.09.2015 DE 102015218958
(43) Veröffentlichungstag der Anmeldung: 26.04.2017
(73) Patentinhaber: Geuder AG, 69126 Heidelberg (DE)
(72) Erfinder: HATTENBACH, Lars-Olof, 67061 Ludwigshafen (DE)
(74) Vertreter: Patent- und Rechtsanwälte Ullrich & Naumann
(86) Internationale Anmeldenummer: PCT/DE2016/200198
(87) Internationale Veröffentlichungsnummer: WO 2016/202332

(56) Entgegenhaltungen:
- WO-A1-2006/090139
- WO-A1-2013/047473
- WO-A1-2014/210586
- DE-A1-102009 032 186
- DE-A1-102010 022 403
- US-A1- 2009 076 463
- US-A1- 2012 172 668

## Beschreibung

Die Erfindung betrifft eine Vorrichtung zum Einführen eines Mediums oder eines Instruments in den menschlichen Körper, insbesondere Port oder Trokar für das menschliche Auge, mit einem Anschlusskörper, einem im Anschlusskörper ausgebildeten Durchgang und einer sich an den Durchgang anschließenden Sonde, wobei die Sonde zum Einführen in den Körper und der Anschlusskörper auf der der Sonde zugewandten Seite zur Anlage außerhalb des Körpers und auf der der Sonde abgewandten Seite zum Einführen bzw. Ankoppeln eines Schlauchs, Instruments oder dgl. dient. Der Anschlusskörper weist eine sich vom Randbereich bis zum Durchgang bzw. zur Sonde erstreckende Ausnehmung oder Aussparung auf, die sich zum Durchgang hin, d.h. in radialer Richtung, verjüngt.

Vorrichtungen der gattungsbildenden Art, insbesondere zur Anwendung in der Ophthalmologie, sind unter dem Begriff Incisionsvorrichtung, Port oder Trokar hinlänglich aus der Praxis bekannt. Lediglich beispielhaft sei dazu auf die EP 1 886 653 A1 verwiesen.

Grundsätzlich geht es hier um eine Vorrichtung zum Einführen eines Mediums oder eines Instruments in den menschlichen Körper. Trokare sind hier lediglich beispielhaft genannt. Die Erfindung ist jedoch keineswegs auf Trokare eingeschränkt. Diese werden nachfolgend lediglich beispielhaft erörtert.

Wie bereits zuvor ausgeführt, handelt es sich bei der hier in Rede stehenden Vorrichtung um ein chirurgisches Instrument, welches beispielsweise für chirurgische Eingriffe im Auge eingesetzt wird. Im Rahmen entsprechender Eingriffe in das menschliche Auge werden regelmäßig mehrere Trokare durch die Sklera hindurch in das Auge eingeführt. Üblicherweise wird ein als Führungsrohr dienender erster Trokar mittels besonderer Lanze derart in das Auge eingesetzt, dass die Spitze der Sonde des Trokars bis in den Glaskörperraum des Auges reicht. Das von der Sonde abgewandte Ende des Trokars, das heißt der Anschlusskörper, dient auf der der Sonde abgewandten Seite zum Anschließen eines Schlauchs, über den das Innere des Auges beispielsweise mit einer Infusionsflüssigkeit versorgbar ist. Dieser Trokar wird üblicherweise auch als Infusionstrokar bezeichnet.

Des Weiteren ist es üblich, ein oder zwei weitere Trokare in das Auge einzusetzen, über die die benötigten Instrumente, beispielsweise Ultraschallschneidinstrumente oder Beleuchtungseinrichtungen, in das Auge einführbar sind. Diese Trokare werden üblicherweise als Instrumententrokare oder - entsprechend der Anwendung - als Beleuchtungstrokare bezeichnet.

Die hier in Rede stehenden Trokare haben den Vorteil, dass diese in eine kleine Öffnung der Sklera eingesetzt werden, wobei die Instrumente durch den jeweiligen Trokar hindurch austauschbar sind. Eine weiterreichende Reizung oder Beschädigung der Sklera wird durch Verwendung des Trokars vermieden.

Aus der DE 10 2010 022 403 A1 ist eine gattungsbildende Vorrichtung bekannt, nämlich ein sogenannter Flachkopftrokar, bei dem der Anschlusskörper äußerst flach ausgebildet ist. Zum zielgerichteten Einführen eines Instruments ist der Anschlusskörper mit einer trichterartigen Öffnung ausgestattet, die im Sinne einer Einführhilfe zu verstehen ist. Der Anschlusskörper mündet über einen Durchgang in die Sonde, wobei zur Vermeidung des Heraustretens von Flüssigkeit aus dem Auge eine Membrane im Durchgang vorgesehen ist, die beim Einführen eines Instruments durchstoßen wird. Die Vorkehrung der Membrane ist konstruktiv aufwändig und verursacht nicht unerhebliche Kosten. Außerdem erfordert das zielgerichtete Durchstoßen der Membrane eine gewisse Übung des Operateurs. Außerdem besteht die Gefahr, dass dort vorgesehene Sollbruchstellen aufgrund fertigungstechnischer Schwankungen/Toleranzen nicht immer gleich ausgebildet sind, wodurch die Handhabung erschwert ist.

Aus der US 2009/0076436 A1, auf welcher der Oberbegriff von Anspruch 1 beruht, ist ein Trokar für das menschliche Auge mit einer seitliche Öffnung bekannt.

Die WO 2006/090139 A1 zeigt ein Instrument zur Implantation eines Sensors in den menschlichen Körper, wobei das Instrument eine seitliche Öffnung aufweist. Aus der US 2012/0172668 A1 ist ein in der Länge verstellbares Kanülensystem zur Inzision in den menschlichen Körper bekannt.

Die WO 2014/210586 A1 zeigt eine Sicherheitskanüle mit einer Sonde zum Einführen in den Körper, wobei der Sonde abstehende Stege zur sicheren Verankerung zugeordnet sind.

Aus der DE 10 2009 032 186 A1 ist eine Vorrichtung zum Einführen eines Mediums oder eines Instruments in den menschlichen Körper, insbesondere in das menschliche Auge, mit einer Sonde zum Einführen in den Körper bekannt, wobei die Sonde zumindest bereichsweise aus flexiblem Material besteht.

Die WO 2013/047473 A1 zeigt einen Trokar mit einem geschlossenen Anschlusskörper und einer geschlossenen Sonde.

Der vorliegenden Erfindung liegt die Aufgabe zugrunde, eine Vorrichtung der gattungsbildenden Art derart auszugestalten und Weiterzubilden, dass bei einfachster Konstruktion eine sichere Handhabung möglich ist.

Voranstehende Aufgabe ist durch eine Vorrichtung mit den Merkmalen des Anspruchs 1 gelöst. Danach ist die gattungsbildende Vorrichtung dadurch gekennzeichnet, dass sich die Ausnehmung (4) über einen Winkel in einem Bereich von etwa 60° erstreckt.

Es ist erkannt worden, dass man von der im Stand der Technik etablierten Vorkehrung einer inneren Dichtung in Form einer Membrane abweichen kann, ohne auf die grundsätzlich erforderliche Abdichtung zu verzichten. So ist erkannt worden, dass man insbesondere beim Einsatz im menschlichen Auge das Gewebe (d.h. das Augengewebe bzw. die Sklera) aktiv als Bestandteil der Trokardichtung nutzen kann. Dazu ist eine ganz besondere konstruktive Maßnahme erforderlich, die ausgehend von einem herkömmlichen Trokar realisiert ist. Die Erfindung geht von einem konventionellen Flachkopftrokar aus, der in üblicher Weise einen Anschlusskörper aufweist. Es ist eine Ausnehmung oder Aussparung im Sinne einer Freifläche vorgesehen, die sich vom Randbereich des Anschlusskörpers bis hin zum Durchgang bzw. zur Sonde oder in die Sonde hinein erstreckt. Diese Ausnehmung oder Aussparung verjüngt sich in radialer Richtung, d.h. zum Durchgang hin. Entsprechend bildet die Ausnehmung oder Aussparung eine Art radialen Trichter im Sinne einer Einführhilfe.

Wird der Trokar bei minimaler Incision gesetzt, nimmt das Augengewebe weitestgehend den durch die Freifläche bzw. die Ausnehmung/Aussparung gebildeten Raum ein und bildet dabei eine Art "in situ"-Dichtung, ohne dass zusätzliche Abdichtmaßnahmen am Trokar vorgesehen sind. Durch Vorkehrung der Ausnehmung/Aussparung erübrigt sich die Vorkehrung einer inneren Abdichtung, wie sie im gattungsbildenden Stand der Technik in Form einer mit Sollbruchstellen ausgestatteten Membrane vorgesehen ist.

Die Ausnehmung/Aussparung hat obendrein den Vorteil, dass das Einführen eines Instruments erleichtert ist, da nämlich die Ausnehmung/Aussparung eine Art radialen Trichter bildet, entlang dessen Wandung das Instrument bis in den Durchgang geführt wird, nämlich durch die so vorgesehenen Wandungen, die die Ausnehmung/Aussparung bilden. In der Mitte angekommen, lässt sich das distale Ende des Instruments durch den Durchgang in die Sonde und von dort in den relevanten Bereich des Körpers bzw. des Auges führen. Während dieses Vorgangs wird das Augengewebe durch das Instrument selbst verdrängt und wird der Port geöffnet, genauso, wie im Stand der Technik die Membrane durchstoßen wird.

Wird das Instrument wieder aus dem Port herausgezogen, dringt das Augengewebe in den durch die Ausnehmung/Aussparung geschaffenen Raum und bildet erneut die erforderliche Abdichtung, so dass der Trokar zur weiteren Verwendung im Auge verbleiben kann, bei gleichzeitiger abdichtender Wirkung zwischen dem Inneren des Auges und der Umgebung. Auf verblüffend einfache Weise ist ein Trokar mit hinreichend guter abdichtender Wirkung geschaffen, ohne die Vorkehrung einer konstruktiv aufwändigen Dichtung bzw. Membrane.

An dieser Stelle sei angemerkt, dass eine hinreichend große Ausnehmung/ Aussparung es ermöglicht, den Trokar universell auszugestalten, nämlich zur Anwendung unterschiedlicher Instrumente, d.h. zur Anwendung von Instrumenten mit unterschiedlichen Durchmessern. Je kleiner man die Ausnehmung/Aussparung gestaltet, desto eher ist es erforderlich, entsprechende Trokare mit unterschiedlichen Größen bzw. Durchmessern im Durchgang und in der Sonde zur Verfügung zu stellen.

Der erfindungsgemäße Trokar nutzt eine raffinierte konstruktive Ausgestaltung in Kombination mit einer Gewebeeigenschaft, wonach das Augengewebe dazu tendiert, nach einer anfänglichen Verdrängung nach dem Anlegen der Incision einen offenen Raum einzunehmen und dabei eine abdichtende Maßnahme zu generieren.

In vorteilhafter Weise ist der Anschlusskörper, d.h. der Kopf des Trokars, scheibenartig, tellerartig oder knopfartig ausgeführt. Entsprechend kann die Ausnehmung die Form eines Kreissegments haben. Auch ist es von Vorteil, wenn sich die Ausnehmung nicht nur über den Anschlusskörper bzw. Kopf hinweg erstreckt, vielmehr auch in die Sonde hinein und vorzugsweise durch die Sonde hindurch bzw. über deren gesamte Länge hinweg ausgebildet ist. Entsprechend ist der Trokar komplett geöffnet, nämlich durch eine Ausnehmung oder Aussparung über seine gesamte Länge hinweg ausgestattet, beginnend vom Anschlusskörper, über den Durchgang hinweg bis ans distale Ende der Sonde. Dies begünstigt die abdichtende Eigenschaft des Augengewebes, insbesondere dadurch, dass diese über die gesamte Länge des Trokars hinweg in den so geschaffenen Raum abdichtend eindringen kann. Beim Einführen eines Instruments, beispielsweise eines Lichtleiters, lässt sich das Augengewebe mühelos verdrängen, wodurch mit Einführung des Instruments die abdichtende Wirkung durch Verdrängung des Augengewebes aufgehoben wird. Wird das Instrument bzw. der Lichtleiter wieder herausgezogen, dringt das Augengewebe in den dabei entsprechenden Freiraum und übernimmt erneut die abdichtende Wirkung zwischen dem Augeninneren und der Umgebung im Sinne einer Dichtung.

Insbesondere unter dem Aspekt einer universell verwendbaren Vorrichtung sollte die Ausnehmung möglichst groß ausgeführt sein, nämlich über einen recht großen Winkel hinweg, wobei die verbleibende Innenwandung zur Führung des Instruments hinreichend groß ist. So ist es von Vorteil, wenn die Ausnehmung über einen Winkel im Bereich von 45° bis 270° ausgeführt ist. Ein Winkel > 180°mach dann Sinn, wenn eine zylinderförmige Sonde mit weniger als 50% umschlossen werden soll. Liegt der Schwerpunkt bei einer guten Führung der Sonde, mit weiterreichender Umschließung, eignet sich ein Winkel im Bereich von 45° bis 180°, um nämlich eine sichere Führung des Instruments beim Einstecken zu gewährleisten. Erfindungsgemäß erstreckt sich die Ausnehmung über einen Winkel im Bereich von etwa 60°, wodurch sich eine hinreichend gute Abdichtung durch das Augengewebe ergibt, wobei die Innenwandung zur hinreichend guten Führung des Instruments ausreicht. Eine universelle Verwendbarkeit zum Einführen unterschiedlicher Instrumente ist ebenfalls gewährleistet.

In Bezug auf den Anschlusskörper ist es von weiterem Vorteil, wenn dieser anschlussseitig, d.h. auf der Oberseite, eine konvexe Grundform hat, nämlich entsprechend nach außen gewölbt ist. Anlageseitig, d.h. auf der Unterseite des Anschlusskörpers, sollte dieser eben bzw. flach ausgebildet sein oder zumindest eine leichte konkave Wölbung haben, nämlich zur besseren Anlage an die Oberfläche des Auges.

In der konvexen Grundform des Anschlusskörpers kann ein mittiger Trichter oder eine Mulde ausgebildet sein, wobei der Trichter bzw. die Mulde in den Durchgang zur Sonde mündet. Der Trichter oder die Mulde ist entsprechend der Ausgestaltung der Ausnehmung ebenfalls unterbrochen bzw. weist eine entsprechende Ausnehmung/Aussparung auf. Der Trichter oder die Mulde dient ebenfalls als Einführhilfe im Sinne der voranstehenden Ausführungen.

Grundsätzlich ist es denkbar, dass im Durchgang eine Nut oder eine Wulst zur Bildung eines Snap-in-Mechanismus mit dem anzuschließenden Schlauch, Instrument oder dergleichen ausgebildet ist. Im Rahmen einer solchen Vorkehrung ist es erforderlich, dass die Ausnehmung/Aussparung über einen Bereich < 180° ausgeführt ist, um nämlich einen hinreichend guten Halt für den Schlauch bzw. das Instrument durch einen zumindest geringfügigen Umgriff zu schaffen. Im Rahmen einer Ausgestaltung der Ausnehmung/Aussparung in einem Bereich von etwa 60° lässt sich eine solche Maßnahme problemlos realisieren.

Entsprechend den voranstehenden Ausführungen dient die Sonde zum Einführen in den Körper bzw. in das Auge. Dabei ist es von Vorteil, wenn die Sonde in einem sich an den Anschlusskörper angrenzenden ersten Bereich eine im Wesentlichen glatte Wandung aufweist, nämlich zum schonenden Sitz in der im Auge angelegten Incision. Dieser Bereich kann sich über eine Strecke von 0,1 bis 0,4 mm hinweg erstrecken, beispielsweise über 0,3 mm hinweg. An diesen ersten Bereich schließt sich ein zweiter Bereich an, nämlich ein Fixierungsbereich, beispielsweise über 1,0 bis 1,5 mm hinweg, insbesondere über einen Bereich von 1,3 mm hinweg. Dieser Bereich hat eine raue, genoppte oder im Wesentlichen quer oder schräg zur Längsachse der Sonde gewellte bzw. geriefte Oberfläche. Auch ist es denkbar, dass die Sonde als Drehteil ausgeführt ist, so dass der Fixierungsbereich Drehriefen auf der Oberfläche hat. Im Rahmen einer weiteren Alternative ist es denkbar, dass der Fixierungsbereich durch ein Außengewinde der Sonde gebildet ist. Wesentlich ist jedenfalls, dass der Fixierungsbereich aufgrund seiner Oberflächenbeschaffenheit, insbesondere einer gewissen Rautiefe, eine im Gewebe fixierende Wirkung hat.

Es ist von weiterem Vorteil, wenn sich an den Fixierungsbereich ein dritter Bereich anschließt, beispielsweise über 1,0 bis 2,0 mm hinweg. Dieser Bereich hat, ähnlich dem ersten Bereich, eine glatte Wandung und begünstigt das Einstecken in die Incision. Dabei ist es von Vorteil, wenn sich die Sonde zum freien Ende hin im Außenquerschnitt in zumindest geringem Maße verjüngt und/oder sich in der Wandstärke reduziert, wobei die Verjüngung durch Reduktion der Wandstärke erreichbar ist. Das Einstecken der Vorrichtung bzw. des Trokars ist dadurch begünstigt.

Der Anschlusskörper und die Sonde können einteilig aus Kunststoff oder Metall hergestellt sein. Im Rahmen einer Fertigung aus Metall bietet es sich an, biokompatible Werkstoffe zu verwenden, beispielsweise Titan oder Titanlegierungen.

Als zusätzliche abdichtende Maßnahme kann eine über die Sonde aufschiebbare, unmittelbar an der Unterseite des Anschlusskörpers zur Anlage bringbare Dichtscheibe vorgesehen sein, wobei diese nicht zwingend erforderlich ist. Die Anbringung einer Dichtscheibe an der Unterseite des Anschlusskörpers unterscheidet sich grundsätzlich von der Vorkehrung einer auf oder im Anschlusskörper angebrachten bzw. vorgesehenen inneren Dichtung mit Sollbruchstelle, wie sie im Stand der Technik bekannt ist.

Eine solche Dichtscheibe kann aus Silikon gefertigt sein. Der Durchmesser der Dichtscheibe entspricht maximal dem Durchmesser des Anschlusskörpers. Es ist denkbar, dass die zusätzliche Dichtscheibe radial geschlitzt bzw. mit einer entsprechenden Ausnehmung ausgestattet ist, ähnlich wie der Anschlusskörper.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein Ausführungsbeispiel eines erfindungsgemäßen Trokars, perspektivisch, von der Oberseite des Anschlusskörpers her und
- Fig. 2.: in einer schematischen Ansicht ein Ausführungsbeispiel eines erfindungsgemäßen Trokars, perspektivisch, von der Unterseite des Anschlusskörpers her.

Bei der in den Figuren gezeigten erfindungsgemäßen Vorrichtung handelt es sich um einen Trokar, der mittels Lanze durch die Sklera hindurch in das menschliche Auge eingesetzt wird.

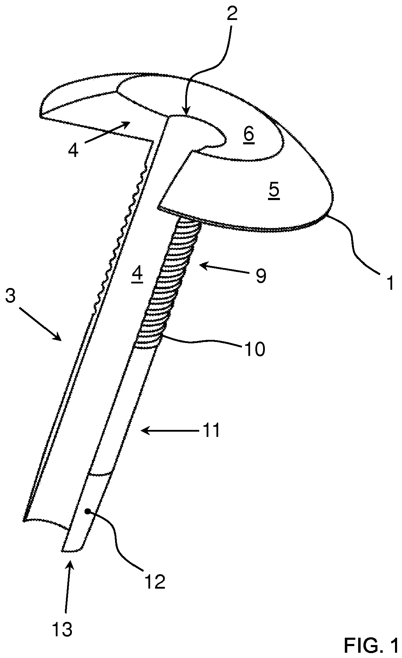

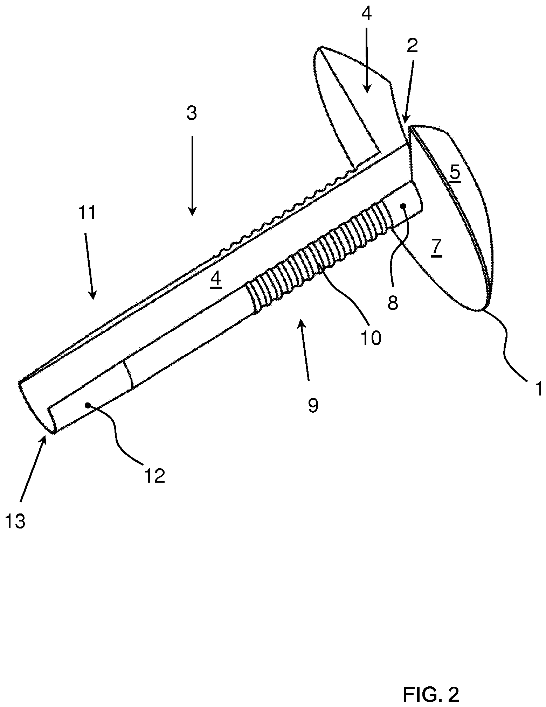

Der in den Fig. 1 und 2 - schematisch - gezeigte Trokar, ein Ausführungsbeispiel einer erfindungsgemäßen Vorrichtung, umfasst einen Anschlusskörper 1, einen im Anschlusskörper 1 ausgebildeten Durchgang 2 und eine sich an den Durchgang 2 anschließende Sonde 3. Die Sonde 3 dient zum Einstecken in den menschlichen Körper bzw. in das Auge. Der Anschlusskörper 1 dient auf der der Sonde 3 zugewandten Seite zur Anlage außerhalb des Auges, nämlich zur Anlage an der Sklera. Auf der der Sonde 3 abgewandten Seite, d. h. anschlussseitig, dient der Anschlusskörper 1 zum Ankoppeln eines in der Figur nicht gezeigten Schlauchs, zum Anstecken oder Einstecken eines Instruments, etc., wodurch eine Art Port gebildet ist.

Die beiden Fig. zeigen deutlich, dass der Anschlusskörper 1 äußerst flach ausgebildet ist, nämlich nur eine geringe Bauhöhe aufweist. Im Konkreten ist der Anschlusskörper 1 ähnlich einem runden Schraubenkopf gebildet, nämlich scheibenartig, tellerartig oder knopfartig. Anschlussseitig ist der Anschlusskörper 1 abgerundet und anlageseitig flach ausgebildet, wobei der Anschlusskörper 1 anlageseitig auch gewölbt oder konkav ausgebildet sein kann, nämlich zur Begünstigung der Anlage an der Oberfläche des Auges.

Erfindungsgemäß ist der Trokar sowohl im Anschlusskörper 1 als auch im Durchgang 2 und in der Sonde 3 mit einer Ausnehmung 4 ausgestattet, die im Anschlusskörper 1 im Sinne eines Teilkreissegments ausgeführt ist. Dadurch ist es möglich, dass bereits beim Einstecken des Trokars in das Augengewebe dieses die Ausnehmung 4 ausfüllt und eine Abdichtung aus Gewebe bildet.

Der Anschlusskörper 1 hat auf seiner der Sonde 3 abgewandten Oberseite eine konvex gewölbte Grundform 5, in der eine konkave Wölbung 6 im Sinne einer Einführhilfe ausgebildet ist. Der gesamte Anschlusskörper 1 ist durch die Ausnehmung 4 unterbrochen, nicht nur die gewölbte Grundform 5 sondern auch die konkave Wölbung 6.

Auf der Unterseite, nämlich auf der Seite der Sonde 3, hat der Anschlusskörper 1 eine flache bzw. ebene Fläche 7, die zur besseren Anpassung an die Oberfläche des Auges konkav ausgeführt sein kann.

Der Anschlusskörper 1 mündet über den Durchgang 2 in die Sonde 3, durch die hindurch ein Instrument, beispielsweise ein Lichtleiter, in den Körper einführbar ist.

Die Sonde 3 schließt sich an die Unterseite des Anschlusskörpers 1 an, bei dem in den Figuren gezeigten Ausführungsbeispiel mit einem kurzen ersten Bereich 8, der an der Oberfläche glatt ausgeführt ist. Durch diese Maßnahme soll das Gewebe im eingesteckten Zustand des Trokars geschont werden.

An den ersten glatten Bereich 8 schließt sich ein zweiter Bereich 9 an, der mit Drehriefen 10 oder mit einem Außengewinde versehen ist. Durch diese Maßnahme soll ein unbeabsichtigtes Herausgleiten des Trokars aus dem Augengewebe vermieden werden.

An den zweiten Bereich 9 schließt sich ein dritter Bereich 11 mit abermals glatter Oberfläche an, der sich zum distalen Ende 13 hin verjüngt. Dieser verjüngte Bereich 12 ist dadurch geschaffen, dass die Wandung zum distalen Ende 13 hin in der Dicke abnimmt, nämlich von außen nach innen gerichtet, so dass das distale Ende 13 der Sonde 3 das Einführen in die Inzision begünstigt. Das distale Ende 13 kann auch abgeschrägt ausgebildet sein, so dass die von der Ausnehmung 4 abgewandte Wandung der Sonde 3 ein mehr oder weniger spitz zulaufendes distales Ende bildet. Dieses Ende kann auch einen Radius mit einer Abrundung aufweisen. Dieser Bereich hat eine glatte Oberfläche, mit möglichst sehr guter Gleiteigenschaft gegenüber dem Augengewebe. Dieser Bereich kann auch schneidend ausgebildet sein, so dass der Einsatz einer Lanze entfallen kann.

Es sei angemerkt, dass die gesamte Vorrichtung, umfassend den Anschlusskörper 1 und die Sonde 3, aus Kunststoff oder Metall gefertigt ist. Bei einer Ausführung aus Metall sind biokompatible Werkstoffe von Vorteil, beispielsweise Titan oder Titanlegierungen.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass das voranstehend beschriebene Ausführungsbeispiel der erfindungsgemäßen Vorrichtung lediglich zur Erörterung der beanspruchten Lehre dient, diese jedoch nicht auf das Ausführungsbeispiel einschränkt.

### Bezugszeichenliste

- 1: Anschlusskörper
- 2: Durchgang
- 3: Sonde
- 4: Ausnehmung/Aussparung
- 5: gewölbte Grundform des Anschlusskörpers (konvex)
- 6: konkave Wölbung (in der gewölbten Grundform)
- 7: flache, ebene Fläche (Unterseite des Anschlusskörpers)
- 8: erster Bereich der Sonde
- 9: zweiter Bereich der Sonde
- 10: Drehriefen, Gewinde
- 11: dritter Bereich der Sonde
- 12: verjüngter Bereich am distalen Ende der Sonde
- 13: distales Ende der Sonde

## Patentansprüche

1. Vorrichtung zum Einführen eines Mediums oder eines Instruments in den menschlichen Körper, insbesondere Port oder Trokar für das menschliche Auge (3), mit einem Anschlusskörper (1), einem im Anschlusskörper (1) ausgebildeten Durchgang (2) und einer sich an den Durchgang (2) anschließenden Sonde (3), wobei die Sonde (3) zum Einführen in den Körper und der Anschlusskörper (1) auf der der Sonde (3) zugewandten Seite zur Anlage außerhalb des Körpers und auf der der Sonde (3) abgewandten Seite zum Einführen bzw. Ankoppeln eines Schlauchs, Instruments oder dergleichen dient, wobei der Anschlusskörper (1) eine sich vom Randbereich bis zum Durchgang (2) bzw. zur Sonde (3) erstreckende Ausnehmung oder Aussparung (4) aufweist, die sich zum Durchgang (2) hin verjüngt
**dadurch gekennzeichnet, dass** sich die Ausnehmung (4) über einen Winkel in einem Bereich von etwa 60° erstreckt.

2. Vorrichtung nach Anspruch 1, **dadurch gekennzeichnet, dass** der Anschlusskörper (1) scheibenartig, tellerartig oder knopfartig als flacher, kreisförmiger Kopf ausgeführt ist und dass die Ausnehmung (4) die Form eines Kreissegments hat.

3. Vorrichtung nach Anspruch 1 oder 2, **dadurch gekennzeichnet, dass** sich die Ausnehmung (4) in die Sonde (3) hinein und vorzugsweise durch die Sonde (3) hindurch bzw. über deren gesamte Länge hinweg erstreckt.

4. Vorrichtung nach einem der Ansprüche 1 bis 3, **dadurch gekennzeichnet, dass** der Anschlusskörper (1) anschlussseitig, d.h. auf der Oberseite, eine konvexe Grundform (5) hat und anlageseitig, d.h. auf der Unterseite, eben/flach oder zumindest leicht konkav ausgebildet ist.

5. Vorrichtung nach Anspruch 4, **dadurch gekennzeichnet, dass** im Anschlusskörper (1) in der konvexen Grundform (5) ein vorzugsweise mittiger Trichter oder eine Mulde ausgebildet ist, der/die in den Durchgang (2) mündet, wobei der Trichter oder die Mulde durch die Ausnehmung (4) unterbrochen ist.

6. Vorrichtung nach einem der Ansprüche 1 bis 5, **dadurch gekennzeichnet, dass** im Durchgang (2) eine Nut oder eine Wulst zur Bildung eines Snap-in-Mechanismus mit dem anzuschließenden Schlauch, Instrument oder dergleichen ausgebildet ist.

7. Vorrichtung nach einem der Ansprüche 1 bis 6, **dadurch gekennzeichnet, dass** die Sonde (3) in einem sich an den Anschlusskörper (1) angrenzenden ersten Bereich (8), vorzugsweise über 0,1 bis 0,4 mm hinweg, eine im Wesentlichen glatte Wandung, in einem sich anschließenden zweiten Bereich (9), nämlich in einem Fixierungsbereich, vorzugsweise über 1,0 bis 1,5 mm hinweg, eine raue, genoppte oder im Wesentlichen quer oder schräg zur Längsachse gewellten bzw. gerieften Oberfläche aufweist,
oder dass zumindest die Sonde (3) als Drehteil ausgeführt ist und der Fixierungsbereich durch Drehriefen (10) gebildet ist,
oder dass der Fixierungsbereich durch ein Außengewinde der Sonde (3) gebildet ist,
und dass ggf. ein sich an den Fixierungsbereich anschließender dritter Bereich (11), vorzugsweise über 1,0 bis 2,0 mm hinweg, mit glatter Wandung genau wie oder ähnlich wie der erste Bereich (8) ausgebildet ist.

8. Vorrichtung nach einem der Ansprüche 1 bis 7, **dadurch gekennzeichnet, dass** sich die Sonde (3) zum freien Ende hin im Außenquerschnitt verjüngt und/oder in der Wandstärke reduziert.

9. Vorrichtung nach einem der Ansprüche 1 bis 8, **dadurch gekennzeichnet, dass** der Anschlusskörper (1) und die Sonde (3) einteilig aus Kunststoff oder Metall, vorzugsweise aus Titan, gefertigt sind.

10. Vorrichtung nach einem der Ansprüche 1 bis 9, **gekennzeichnet durch** eine über die Sonde (3) aufschiebbare, unmittelbar an der Unterseite des Anschlusskörpers zur Anlage bringbare Dichtscheibe, vorzugsweise aus Silikon, wobei der Durchmesser der Dichtscheibe maximal dem Durchmesser des Anschlusskörpers (1) entspricht.

## Claims

1. Device for introducing a medium or an instrument into the human body, in particular port or trocar for the human eye (3), having a connection member (1), a passage (2) which is formed in the connection member (1) and a probe (3) which connects to the passage (2), wherein the probe (3) is used for introduction into the body and the connection member (1) at the side facing the probe (3) is used for abutment outside the body and at the side facing away from the probe (3) is used for introducing or coupling a hose, instrument or the like, wherein the connection member (1) has a recess or indentation (4) which extends from the edge region as far as the passage (2) or the probe (3) and which tapers in the direction towards the passage (2),
**characterised in that** the recess (4) extends over an angle in a range of approximately 60°.

2. Device according to claim 1, **characterised in that** the connection member (1) is constructed in a disc-like, platelike or knob-like manner as a flat, circular head and **in that** the recess (4) is in the form of a circle segment.

3. Device according to claim 1 or 2, **characterised in that** the recess (4) extends into the probe (3) and preferably through the probe (3) or over the entire length thereof.

4. Device according to any one of claims 1 to 3, **characterised in that** the connection member (1) at the connection side, that is to say, at the upper side, has a convex basic shape (5) and at the abutment side, that is to say, at the lower side, is constructed in a planar/flat manner or at least in a slightly concave manner.

5. Device according to claim 4, **characterised in that** there is formed in the connection member (1) in the convex basic shape (5) a preferably central funnel or a hollow which opens in the passage (2), wherein the funnel or the hollow is interrupted by the recess (4).

6. Device according to any one of claims 1 to 5, **characterised in that** a groove or a bead for forming a snap-in mechanism with the hose, instrument or the like which is intended to be connected is formed in the passage (2).

7. Device according to any one of claims 1 to 6, **characterised in that** the probe (3) in a first region (8) adjacent to the connection member (1), preferably over from 0.1 to 0.4 mm, has a substantially smooth wall, in an adjacent second region (9), that is to say, in a fixing region, preferably over from 1.0 to 1.5 mm, has a surface which is rough, burled or undulating or grooved substantially transversely or obliquely relative to the longitudinal axis, or **in that** at least the probe (3) is constructed as a rotary component and the fixing region is formed by means of tool marks (10),
or **in that** the fixing region is formed by means of an outer thread of the probe (3),
and **in that** where applicable a third region (11) which is adjacent to the fixing region, preferably over from 1.0 to 2.0 mm, is constructed with a smooth wall precisely in the same manner as or similar to the first region (8).

8. Device according to any one of claims 1 to 7, **characterised in that** the probe (3) tapers in the direction towards the free end in the outer cross-section and/or reduces in terms of the wall thickness.

9. Device according to any one of claims 1 to 8, **characterised in that** the connection member (1) and the probe (3) are produced in one piece from plastics material or metal, preferably from titanium.

10. Device according to any one of claims 1 to 9, **characterised by** a sealing disc which can be pushed over the probe (3) and which can be brought into abutment directly at the lower side of the connection member and which is preferably produced from silicone, wherein the diameter of the sealing disc corresponds to a maximum of the diameter of the connection member (1).

## Revendications

1. Dispositif pour l'introduction d'un fluide ou d'un instrument dans le corps humain, plus particulièrement une connexion ou un trocart pour l'oeil humain (3) avec un corps de raccordement (1), un passage (2) réalisé dans le corps de raccordement (1) et une sonde (3) se raccordant au passage (2), la sonde (3) étant destinée à être introduite dans le corps et le corps de raccordement (1) permettant un appui à l'extérieur du corps et, sur le côté opposé à la sonde (3), l'introduction ou le couplage d'un tuyau, d'un instrument ou autre, le corps de raccordement (1) comprenant une cavité ou une encoche (4), s'étendant du bord jusqu'au passage (2) ou jusqu'à la sonde (3), qui se rétrécit en direction du passage (2),
**caractérisé en ce que** la cavité (4) s'étend sur un angle dans une zone d'environ 60°.

2. Dispositif selon la revendication 1, **caractérisé en ce que** le corps de raccordement (1) est conçu sous la forme d'un disque, d'une plaque ou d'un bouton, comme une tête plate circulaire et **en ce que** la cavité (4) présente la forme d'un segment circulaire.

3. Dispositif selon la revendication 1 ou 2, **caractérisé en ce que** la cavité (4) s'étend à l'intérieur de la sonde (3) et de préférence à travers la sonde (3) ou sur toute sa longueur.

4. Dispositif selon l'une des revendications 1 à 3, **caractérisé en ce que** le corps de raccordement (1) présente, du côté du raccordement, c'est-à-dire sur le côté supérieur, une forme de base convexe (5) et présente, du côté de l'installation, c'est-à-dire sur le côté inférieur, une forme plane/plate ou au moins légèrement concave.

5. Dispositif selon la revendication 4, **caractérisé en ce que**, dans le corps de raccordement (1), dans la forme de base convexe (5), est réalisé un entonnoir ou un creux, de préférence central, qui débouche dans le passage (2), l'entonnoir ou le creux étant interrompu par la cavité (4).

6. Dispositif selon l'une des revendications 1 à 5, **caractérisé en ce que**, dans le passage (2) est réalisée une rainure ou un bourrelet pour la formation d'un mécanisme d'encliquetage avec le tuyau, l'instrument ou autre à raccorder.

7. Dispositif selon l'une des revendications 1 à 6, **caractérisé en ce que** la sonde (3) présente, dans une première zone (8) adjacente au corps de raccordement (1), de préférence sur 0,1 à 0,4 mm, une paroi globalement lisse et, dans une deuxième zone (9) qui la prolonge, à savoir dans une zone de fixation, de préférence sur 1,0 à 1,5 mm, une surface rugueuse, à picots ou une surface ondulée ou striée de manière globalement transversale ou oblique par rapport à l'axe longitudinal,
ou **en ce qu'**au moins la sonde (3) est conçue comme une pièce rotative et la zone de fixation est constituée de stries rotatives (10),
ou **en ce que** la zone de fixation est constituée d'un filetage externe de la sonde (3),
et **en ce que**, le cas échéant, une troisième zone (11) prolongeant la zone de fixation, de préférence sur 1,0 à 2,0 mm, avec une paroi lisse, est réalisée exactement ou de manière similaire à la première zone (8).

8. Dispositif selon l'une des revendications 1 à 7, **caractérisé en ce que** la sonde (3) se rétrécit vers l'extrémité libre en section transversale extérieure et/ou son épaisseur de paroi diminue.

9. Dispositif selon l'une des revendications 1 à 8, **caractérisé en ce que** le corps de raccordement (1) et la sonde (3) sont réalisés d'une seule pièce en matière plastique ou en métal, de préférence en titane.

10. Dispositif selon l'une des revendications 1 à 9, **caractérisé par** une bague d'étanchéité pouvant être montée sur la sonde (3) et appuyée directement contre le côté inférieur du corps de raccordement, de préférence en silicone, le diamètre de la bague d'étanchéité correspondant au maximum au diamètre du corps de raccordement (1).
